# EUROPÄISCHE PATENTSCHRIFT

(11) **EP 1 682 480 B1**
(45) Veröffentlichungstag und Bekanntmachung des Hinweises auf die Patenterteilung: **01.02.2012**
(21) Anmeldenummer: 04766552.6
(22) Anmeldetag: 20.08.2004
(51) Int. Cl.: C07C 67/08, C07C 69/78

(54) **VERFAHREN ZUR HERSTELLUNG VON BENZOESÄUREESTERN**
METHOD FOR PRODUCING BENZOIC ACID ESTERS
PROCÉDÉ DE PRODUCTION D'ESTERS DE L'ACIDE BENZOIQUE

(30) Priorität: 10.10.2003 DE 10347863
(43) Veröffentlichungstag der Anmeldung: 26.07.2006
(73) Patentinhaber: Evonik Oxeno GmbH, 45772 Marl (DE)
(72) Erfinder: GRASS, Michael, 45721 Haltern am See (DE); GUBISCH, Dietmar, 45772 Marl (DE); WOELK-FÄHRMANN, Michael, 45768 Marl (DE)
(74) Vertreter: Hirsch, Hans-Ludwig
(86) Internationale Anmeldenummer: PCT/EP2004/051854
(87) Internationale Veröffentlichungsnummer: WO 2005/037764

(56) Entgegenhaltungen:
- WO-A-98/00173
- US-A- 5 898 077
- US-A1- 2004 015 007

## Beschreibung

Die vorliegende Erfindung betrifft ein Verfahren zur Herstellung von Benzoesäureestern aus Benzoesäure und Alkoholen mit 9 oder 13 C-Atomen.

Benzoesäureester, deren Alkoxygruppen 9 oder 13 C-Atome aufweisen, werden als Filmbildehilfsmittel in Kompositionen wie Dispersionsfarben, Mörteln, Putzen, Klebstoffen und Lacken eingesetzt. Weiterhin können sie als Weichmacher und/oder Viskositätserniedriger in Weich-PVC-Anwendungen, vor allem in Plastisolen, eingesetzt werden.

Es ist bekannt, Carbonsäureester durch Umsetzung von Carbonsäuren mit Alkoholen herzustellen. Diese Reaktion kann autokatalytisch oder katalytisch, beispielsweise durch Brønstedt- oder Lewissäuren, durchgeführt werden. Ganz gleich welche Art der Katalyse gewählt wird, es entsteht immer ein temperaturabhängiges Gleichgewicht zwischen den Einsatzstoffen (Carbonsäure und Alkohol) und den Produkten (Ester und Wasser). Um das Gleichgewicht zu Gunsten des Esters zu verschieben, wird bei vielen Veresterungen ein Schleppmittel eingesetzt, mit dessen Hilfe das Reaktionswasser aus dem Reaktionsgemisch entfernt wird. Wenn einer der Einsatzstoffe (Alkohol oder Carbonsäure) niedriger siedet als der gebildete Ester und mit Wasser eine Mischungslücke bildet, kann dieses Edukt als Schleppmittel verwendet und nach Abtrennung vom Wasser wieder in den Ansatz zurückgeführt werden. Bei der Veresterung von Carbonsäuren wird häufig der eingesetzte Alkohol als Schleppmittel eingesetzt.

Für viele Anwendungszwecke müssen die durch Veresterung einer Carbonsäure hergestellten Ester eine niedrige Säurezahl besitzen, d. h. die Umsetzung der Carbonsäure sollte praktisch quantitativ erfolgen. Anderenfalls wird die Ausbeute gemindert und die Säure muss, beispielsweise durch Neutralisation, abgetrennt werden. Dies ist aufwändig und kann zu Nebenprodukten führen, die entsorgt werden müssen. Um einen möglichst hohen Umsatz der Carbonsäure zu erhalten, werden Veresterungen in der Regel mit einem Alkoholüberschuss durchgeführt.

Als Veresterungskatalysatoren können Säuren, wie beispielsweise Schwefelsäure oder p-Toluolsulfonsäure, oder Metalle und deren Verbindungen eingesetzt werden. Geeignet sind z. B. Zinn, Titan, Zirkonium, die als feinverteilte Metalle oder zweckmäßig in Form ihrer Salze, Oxide oder löslichen organischen Verbindungen verwendet werden. Die Metallkatalysatoren sind im Gegensatz zu Protonensäuren Hochtemperaturkatalysatoren, die ihre volle Aktivität erst bei Temperaturen oberhalb 180 °C erreichen. Sie werden jedoch in der Technik bevorzugt eingesetzt, weil sie im Vergleich zur Protonenkatalyse weniger Nebenprodukte, wie beispielsweise Olefine aus dem eingesetzten Alkohol, bilden. Beispielhafte Vertreter für Metallkatalysatoren sind Titansäureester wie Tetraisopropylorthotitanat oder Tetrabutylorthotitanat sowie Zirconiumester wie Tetrabutylzirconat.

Nach der erfolgten Veresterung müssen der überschüssige Einsatzalkohol, Säurereste, der Katalysator und/oder seine Folgeprodukte sowie sonstige Nebenprodukte vom gewünschten Carbonsäureester getrennt werden. Die Wirtschaftlichkeit eines Veresterungsverfahren ist nur dann gut, wenn sowohl der technische Aufwand als auch der Zeitaufwand für die Stofftrennung gering sind.

Die Veresterung von Carbonsäuren mit Zinnverbindungen wird in den Patentschriften GB 2 098 211, EP 0 036 712 und EP 0 037 172 dargelegt. Dabei wird ein Teil der Zinnverbindungen auf ein Metallgeflecht aufgezogen, das mit der Reaktionslösung in Kontakt steht, der andere Teil der Zinnverbindungen ist in der Reaktionslösung gelöst und/oder suspendiert. Die Veresterung erfolgt unter Abdestillieren des Reaktionswassers. Nach der Veresterung werden in einem zweiten Gefäß noch vorhandene Säuren neutralisiert und deren Salze und Zinnverbindungen abgetrennt. Dies geschieht dadurch, dass zu dem Rohester eine feste Base zugegeben wird und die ausgefallenen Feststoffe (Base, Salze, Zinnverbindungen) abfiltriert werden oder der Rohester mit einer wässrigen Base extrahiert wird. Bei diesen Aufarbeitungsverfahren entsteht entweder eine wässrige Phase, die anorganische und organische Verbindungen enthält oder ein Feststoffgemisch mit organischen und anorganischen Verbindungen. Die Entsorgung dieser Nebenprodukte bedingt unter Umständen hohe Kosten.

US 6 235 924 beschreibt ein Verfahren zur Herstellung von Benzoesäureestern durch Umsetzung von Benzoesäure mit einem Alkohol in Gegenwart eines Titankatalysators. Die Veresterung wird unter destillativer Abtrennung des Reaktionswassers durchgeführt. Aus dem Estergemisch wird der Benzoesäureester durch fraktionierte Destillation abgetrennt. Nachteilig an diesem Verfahren ist der Zeit- und Energieaufwand für die Destillation.

In US 5,898,077 wird ein Verfahren zur Veresterung von aliphatischen Mono- oder Dicarbonsäuren, aromatischen Polycarbonsäuren und polymerisierten Carbonsäuren mit Alkoholen in Gegenwart eines zinn- oder zinkenthaltenden Veresterungskatalysators beschrieben. Der Aufreinigungsschritt enthält eine Behandlung mit einem Chelatbildner. Die Anwendung des Verfahrens auf Benzoesäure wird nicht in Betracht gezogen.

US 2004/0015007 A1 beschreibt Isononylbenzoate und deren Herstellung, wobei diese weniger als 10 mol-% 3,5,5-Trimethylhexanol als Alkoholkomponente im Ester enthalten.

In WO 89/00173A wird die Verwendung von Benzoesäureestern mit 10 bis 12 Kohlenstoffatomen im Alkoholrest in Farbzusammensetzungen beschrieben. Bei der Aufreinigung der Benzoesäureester ist eine Behandlung mit Base obligatorisch.

Es bestand daher die Aufgabe, ein Verfahren zur Herstellung von Benzoesäureestern zu finden, das die Nachteile der bekannten Verfahren nicht aufweist.

Überraschenderweise wurde gefunden, dass Benzoesäureester von hoher Reinheit durch Umsetzung von Benzoesäure mit Alkoholen mit 9 oder 13 C-Atomen in Gegenwart einer Zinn(II)verbindung einfach hergestellt werden können, wenn die Veresterung unter Abdestillieren des Reaktionswassers erfolgt, nach der Veresterung der im Reaktionsgemisch vorhandene überschüssige Alkohol durch Destillation und Ausstrippen mit einem Gas oder Dampf entfernt wird und ohne eine Behandlung mit einer Base der so erhaltene Rohester filtriert wird. Durch die Filtration kann der Zinnkatalysator und/oder dessen zinnhaltige Folgeprodukte so weit abgetrennt werden, dass der Zinngehalt des Endproduktes (Filtrats) deutlich unter 1 mg/kg (ppm) liegt.

Gegenstand der vorliegenden Erfindung ist deshalb ein Verfahren zur Herstellung von Benzoesäureestern, deren Alkoxygruppen 9 oder 13 Kohlenstoffatome aufweisen, durch Umsetzung von Benzoesäure mit zumindest einem Alkohol, der 9 oder 13 Kohlenstoffatome aufweist, wobei das entstehende Reaktionswasser während der Veresterungsreaktion durch Destillation mit dem im Überschuss eingesetzten Alkohol aus dem Reaktionsgemisch entfernt wird und der bei der Veresterungsreaktion nicht umgesetzte Alkohol nach der Veresterungsreaktion entfernt wird, welches dadurch gekennzeichnet ist, dass die Umsetzung in Gegenwart einer Zinn (II) Verbindung als Katalysator bei einer Temperatur von 160 °C bis 250 °C erfolgt und dass ohne Behandlung mit einer Base aus dem nach der Abtrennung des nicht umgesetzten Alkohols verbleibenden Reaktionsgemisch der Katalysator und/oder dessen Folgeprodukte durch eine Filtration oder durch Zentrifugieren nahezu vollständig abgetrennt werden können.

Das erfindungsgemäße Verfahren zeichnet sich durch folgende Vorteile aus:

Die Aufarbeitung, insbesondere die einfache Abtrennung des Katalysators durch Filtration, ist im Vergleich zu bekannten Verfahren wenig aufwändig.

Durch Verzicht auf die Zugabe einer Base fallen weder mit anorganischen und organischen Stoffen befrachtete Abwässer noch Neutralisationssalze an.

Der abgetrennte Katalysator bzw. dessen Folgeprodukt(e) kann/können einfach aufgearbeitet oder entsorgt werden.

Dadurch, dass keine Basen oder andere Hilfsstoffe dem Reaktionsgemisch zugegeben werden müssen, werden Zeit und Kosten gespart und eine zusätzliche Verunreinigung des Esters vermieden.

Das erfindungsgemäße Verfahren zur Herstellung von Benzoesäureestern, mit dem Verfahren hergestellte Zusammensetzungen sowie deren Verwendungen wird/werden im Folgenden beschrieben, ohne dass die Erfindung auf diese Ausführungsarten beschränkt sein soll.

Das erfindungsgemäße Verfahren zur Herstellung von Benzoesäureestern, deren Alkoxygruppen 9 oder 13 Kohlenstoffatome aufweisen, durch Umsetzung von Benzoesäure mit einem oder mehreren Alkohol(en), die 9 oder 13 Kohlenstoffatome aufweisen, wobei das entstehende Reaktionswasser während der Veresterungsreaktion durch Destillation mit dem im Überschuss eingesetzten Alkohol aus dem Reaktionsgemisch entfernt wird und der bei der Veresterungsreaktion nicht umgesetzte Alkohol nach der Veresterungsreaktion entfernt wird, zeichnet sich dadurch aus, dass die Umsetzung in Gegenwart einer Zinn (II) Verbindung als Katalysator bei einer Temperatur von 160 °C bis 250 °C erfolgt und dass ohne Behandlung mit einer Base aus dem nach der Abtrennung des nicht umgesetzten Alkohols verbleibenden Reaktionsgemisch der Katalysator und/oder dessen Folgeprodukte durch eine Filtration oder durch Zentrifugieren nahezu vollständig abgetrennt werden kann, so dass der Zinngehalt des Endproduktes (des Filtrates) unter 1 mg/kg (ppm) liegt, insbesondere deutlich unter 1 mg/kg liegt und bevorzugt weniger als 0,1 mg/kg beträgt.

Es findet also während der gesamten Veresterungsreaktion sowie der anschließenden Abtrennung von überschüssigem Alkohol und Katalysator keine Behandlung mit einer Base bzw. kein Neutralisationsschritt statt.

Im erfindungsgemäßen Verfahren werden zur Veresterung der Benzoesäure verzweigte oder lineare aliphatische Alkohole oder Gemische von Alkoholen mit 9 oder 13 C-Atomen eingesetzt. Die Alkohole können ein- oder mehrwertig sein, sind bevorzugt aber einwertig. Die Alkohole können sekundär oder primär, linear oder verzweigt sein. Als Alkohol kann ein Gemisch von Alkoholen mit gleicher oder unterschiedlicher Anzahl an Kohlenstoffatomen eingesetzt werden. Die Alkohole können als isomerenreine Verbindung, als Gemisch isomerer Verbindungen oder als Gemisch von isomeren oder isomerenreinen Verbindungen mit unterschiedlicher Anzahl an Kohlenstoffatomen eingesetzt werden.

Die eingesetzten Alkohole können aus verschiedenen Quellen stammen. Geeignete Einsatzalkohole sind beispielsweise Fettalkohole, Alkohole aus dem Alfolprozess (Oligomerisierung von Ethylen in Gegenwart von Aluminiumalkylen mit anschließender Oxidation und Hydrolyse zu den entsprechenden primären Alkoholen) oder Alkohole oder Alkoholgemische, die durch Hydrierung von gesättigten oder ungesättigten Aldehyden gewonnen wurden, insbesondere solchen, deren Synthese einen Hydroformylierungsschritt einschließt.

Alkohole, die im erfindungsgemäßen Verfahren eingesetzt werden, sind Nonanole, und/oder Tridecanole. Besonders bevorzugte Einsatzalkohole sind Gemische isomerer , Nonanole oder Tridecanole, wobei die letzteren aus den entsprechenden Butenoligomeren, insbesondere Oligomeren von linearen Butenen, durch Hydroformylierung und anschließende Hydrierung gewonnen werden können. Die Herstellung der Butenoligomeren kann im Prinzip nach drei Verfahren durchgeführt werden. Die sauer katalysierte Oligomerisierung, bei der technisch z. B. Zeolithe oder Phosphorsäure auf Trägem eingesetzt werden, liefert die verzweigtesten Oligomeren. Bei Einsatz von linearen Butenen entsteht beispielsweise eine C₈-Fraktion, die im Wesentlichen aus Dimethylhexenen besteht (WO 92/13818). Ein ebenfalls weltweit ausgeübtes Verfahren ist die Oligomerisierung mit löslichen Ni-Komplexen, bekannt als DIMERSOL-Verfahren (B. Cornils, W. A. Herrmann, Applied Homogenous Catalysis with Organometallic Compounds, Seite 261-263, Verlag Chemie 1996). Weiterhin kann die Oligomerisierung an Nickel-Festbett-Katalysatoren ausgeübt werden, wie dies beispielsweise der OCTOL-Process beschreibt (Hydrocarbon Process., Int. Ed. (1986) 65 (2. Sect. 1), Seite 31-33).

Ganz besonders bevorzugte Einsatzstoffe für die erfindungsgemäße Veresterung sind Gemische isomerer Nonanole oder Gemische isomerer Tridecanole, die durch Oligomerisierung linearer Butene zu C₈-Olefinen und C₁₂-Olefinen nach dem Octol-Prozess, mit anschließender Hydroformylierung und Hydrierung hergestellt werden.

Als Katalysator werden im erfindungsgemäßen Verfahren eine oder mehrere zweiwertige Zinnverbindungen oder Zinnverbindungen bzw. elementares Zinn, die sich mit den Edukten zu zweiwertigen Zinnverbindungen umsetzen können, eingesetzt. Beispielsweise kann als Katalysator Zinn, Zinn(II)chlorid, Zinn(II)sulfat, Zinn(II)alkoholate oder Zinn(II)salze von organischen Säuren, insbesondere von Mono- und Dicarbonsäuren, verwendet werden. Bevorzugte Zinnkatalysatoren sind Zinn(II)oxalat und/oder Zinn(II)benzoat.

Es hat sich als vorteilhaft erwiesen, wenn zu Beginn der Reaktion vorzugsweise ein molares Verhältnis von Zinn zu Benzoesäure von 10⁻⁵ bis 10⁻³ zu 1, besonders bevorzugt von 10⁻⁴ bis 10⁻³ zu 1 eingestellt wird.

Die Veresterung wird in einem Reaktionsgefäß (Reaktor) durchgeführt, in dem der Reaktionsansatz mit Hilfe eines Rührers oder einer Umlaufpumpe intensiv vermischt werden kann. Die Edukte und der Katalysator können gleichzeitig oder hintereinander in den Reaktor eingefüllt werden. Liegt die Benzoesäure bei der Einfülltemperatur als Feststoff vor, so kann es zweckmäßig sein, den Alkohol oder das Alkoholgemisch vorzulegen. Die Benzoesäure kann als Pulver, Granulat, Kristallisat oder Schmelze eingespeist werden. Ebenso ist es möglich, die Benzoesäure in einem Lösemittel gelöst, vorzugsweise in einem Alkohol und bevorzugt in einem Alkohol, der ebenfalls als Edukt eingesetzt wird, in den Reaktor einzuspeisen. Um die Chargenzeit zu verkürzen, ist es vorteilhaft, während des Einfüllens mit dem Aufheizen zu beginnen. Der Katalysator kann in reiner Form, als Suspension oder als Lösung, bevorzugt in eingesetztem Alkohol oder Alkoholgemisch, zu Beginn oder erst nach Erreichen der Reaktionstemperatur in den Reaktor eingebracht werden. Dabei kann die einzusetzende Katalysatormenge auf einmal oder in mehreren Portionen zugegeben werden.

Der umzusetzende Alkohol, der auch als Schleppmittel dient, wird im stöchiometrischen Überschuss, bevorzugt mit einem Überschuss von 5 bis 50 %, besonders bevorzugt 10 bis 30 % der stöchiometrisch notwendigen Menge eingesetzt.

Die Reaktionstemperaturen liegen im erfindungsgemäßen Verfahren im Bereich von 160 °C bis 250 °C, bevorzugt von 180 °C bis 230 °C, besonders bevorzugt von 190 °C bis 210 °C. Die optimalen Temperaturen hängen von dem/den Einsatzalkohol(en), dem Reaktionsfortschritt, der Katalysatorart und der Katalysatorkonzentration ab. Sie können für jeden Einzelfall durch Versuche leicht ermittelt werden. Höhere Temperaturen erhöhen die Reaktionsgeschwindigkeiten und begünstigen Nebenreaktionen, wie beispielsweise Wasserabspaltung aus Alkoholen oder Bildung farbiger Nebenprodukte. Es ist zur Entfernung des Reaktionswassers erforderlich, dass der Alkohol aus dem Reaktionsgemisch abdestillieren kann. Die gewünschte Temperatur oder der gewünschte Temperaturbereich kann durch den Druck im Reaktionsgefäß (leichter Überdruck-Normaldruck-Unterdruck) eingestellt werden.

Beispielsweise wird bei der Veresterung von Benzoesäure mit einem Gemisch isomerer Nonanole besonders bevorzugt in einem Temperaturbereich von 190 °C bis 210 °C im Druckbereich von 1 bar bis 100 mbar gearbeitet.

Das während der Veresterungsreaktion entstehende Reaktionswasser wird durch Destillation, insbesondere eine azeotrope Destillation während der laufenden Veresterungsreaktion entfernt. Durch diese Maßnahme wird nicht nur das Reaktionswasser, sondern auch ein Teil des Alkohols entfernt. Es ist möglich, die durch die azeotrope Destillation entfernte Flüssigkeitsmenge vollständig oder teilweise zu ersetzen, indem die abgetrennte Flüssigkeit in eine organische Phase, die neben dem Alkohol auch Benzoesäureester enthalten kann, und in eine Wasserphase getrennt wird und die organische Phase in die Veresterungsreaktion zurückgeführt wird. Optional kann frischer Alkohol zur abgetrennten organischen Phase, die in die Reaktion zurückgeführt wird, beigefügt werden.

Vorzugsweise wird die aus dem Reaktionsgemisch durch Destillation bei der Entfernung des Reaktionswassers entfernte Flüssigkeit durch ein entsprechendes Volumen an Flüssigkeit ersetzt, welches dem Reaktionsgemisch wieder zugegeben wird. Die in die Reaktion zurückzuführende Flüssigkeitsmenge kann teilweise oder vollständig aus dem Einsatzalkohol oder Einsatzalkoholgemisch bestehen. Die während der Veresterung durch (azeotrope) Destillation aus dem Reaktionsgemisch entfernte Flüssigkeitsmenge kann z.B. dadurch zumindest teilweise ergänzt werden, dass die abgetrennte Flüssigkeit in eine Wasserphase und eine organische Phase getrennt wird und die organische Phase in die Veresterungsreaktion zurückgeführt wird. Ebenso ist es möglich, dass das Reaktionsgemisch um die während der Veresterung durch (azeotrope) Destillation aus dem Reaktionsgemisch abgetrennte Flüssigkeitsmenge vollständig oder teilweise ergänzt wird, indem die abgetrennte Flüssigkeit in eine Wasserphase und eine organische Phase getrennt wird und die organische Phase, zusätzlich mit frischem Alkohol versetzt, in die Veresterungsreaktion zurückgeführt wird. Weiterhin ist es möglich, die aus dem Reaktionsgemisch durch (azeotrope) Destillation entfernte Flüssigkeitsmenge ganz oder teilweise durch frischen Alkohol, d. h. aus einem in einem Vorratsgefäß bereit stehenden Alkohol zu ersetzen.

Das während der Reaktion als Azeotrop oder mit nahezu azeotroper Zusammensetzung aus der Reaktionsmischung abdestillierte Alkohol-Wasser-Gemisch kann gegebenenfalls auch noch Benzoesäureester aufweisen. Die erhaltenen Brüden verlassen, z. B. über eine kurze Kolonne (Einbauten oder Füllkörper; 1 bis 5, vorzugsweise 1 bis 3 theoretische Böden), das Reaktionsgefäß und werden kondensiert. Das Kondensat kann, z.B. in einem Phasenabscheider oder einem Koaleszer, in eine wässrige und in eine organische Phase getrennt werden. Es kann vorteilhaft sein, das abgetrennte Azeotrop weit unter die Kondensationstemperatur der Brüden abzukühlen, so dass eine bessere Phasentrennung erreicht wird. Die wässrige Phase wird abgetrennt und kann, gegebenenfalls nach Aufarbeitung, verworfen oder als Strippwasser bei der Nachbehandlung des Esters verwendet werden.

Die organische Phase, die nach Trennung des azeotropen Destillats anfällt, kann teilweise oder vollständig in das Reaktionsgefäß zurückgeführt werden. In der Praxis hat sich eine standgeregelte Füllstandskontrolle der Reaktion zur Zufuhr des Alkohols bewährt, wobei bei nicht ausreichender Menge an durch die Destillation anfallendem Alkohol bzw. Alkoholgemisch eine Zugabe von Frischalkohol vorteilhaft ist.

Für die Zufuhr der organischen Phase in die Veresterungsreaktion gibt es verschiedene Möglichkeiten. Die organische Phase kann beispielsweise als Rückfluss auf die Kolonne gegeben werden. Eine andere Möglichkeit ist, die organische Phase, gegebenenfalls nach Aufheizung, in das flüssige Reaktionsgemisch zu pumpen. Durch die Abtrennung des Reaktionswassers sinkt das Reaktionsvolumen in der Apparatur. Es ist jedoch vorteilhaft, wie in DE 100 43 545.9 beschrieben (kürzere Reaktionszeit) während der Reaktion so viel Alkohol nachzuspeisen, wie dem Volumen des abgetrennten Destillats (Wasser und gegebenenfalls Alkohol) entspricht, sodass der Füllstand im Reaktionsgefäß konstant bleibt. Durch die Vergrößerung des Alkoholüberschusses wird das Gleichgewicht zu Gunsten des/der Benzoesäureester(s) verschoben.

Nach Beendigung der Reaktion weist das Reaktionsgemisch Benzoesäureester, überschüssige/n Alkohol(e) und Katalysator und dessen Folgeprodukt(e) sowie ggf. leichtsiedende und/oder schwersiedende Nebenprodukte auf. Vorzugsweise wird die Reaktion so durchgeführt, dass praktisch keine freie Benzoesäure im Reaktionsgemisch mehr enthalten ist, die Benzoesäure also praktisch vollständig verestert ist. Als Maß hierfür dient die Säurezahl nach DIN EN ISO 2114. Das Produkt nach dem erfindungsgemäßen Verfahren weist vorzugsweise eine Säurezahl von < 0,1 mg KOH/g, bevorzugt < 0,07 mg KOH/g, besonders bevorzugt < 0,04 mg KOH/g auf.

Der nicht umgesetzte Alkohol kann durch Strippen, Destillation oder Wasserdampfdestillation oder durch eine Kombination von zwei oder mehr dieser Verfahren entfernt werden. Bevorzugt wird der nicht umgesetzte Alkohol nach der Veresterungsreaktion durch eine Vakuumdestillation und eine anschließende Strippung mit Wasserdampf oder Stickstoff abgetrennt.

Die Abtrennung des Überschussalkohols kann z. B. zunächst durch Vakuumdestillation, optional ohne weitere Energiezufuhr, wahlweise bis zu einer Alkoholkonzentration von unter 10 %, bevorzugt unter 5%, besonders bevorzugt unter 3 %, ganz besonders bevorzugt unter 1 % im Reaktionsgemisch durchgeführt werden. Die Destillationsbedingungen hierfür betragen bezüglich der Temperatur vorzugsweise von 160 bis 250 °C, bevorzugt von 180 bis 230 °C, der Druck beträgt bevorzugt von 5 mbar bis 1 bar. Bei der Herstellung von Isononylbenzoat wird die Vakuumdestillation beispielsweise bevorzugt bei einer Destillationstemperatur von 180 bis 210 °C und einem Destillationsdruck von 10 bis 320 mbar durchgeführt.

Nach der Vakuumdestillation können der restliche Alkohol und gegebenenfalls vorhandene restliche Leichtsieder durch Strippen mit einem inerten Gas, wie beispielsweise Stickstoff oder Wasserdampf im Temperaturbereich von 130 bis 240 °C entfernt werden. Bevorzugt wird mit Wasserdampf im Temperaturbereich von 170 bis 220 °C bei einem Druck von 20 bis 500 mbar, bevorzugt von 20 bis 150 mbar, gestrippt. Druck und Temperatur sind sowohl beim Strippen als auch bei der Vakuumdestillation jeweils so zu wählen, dass der Ester in der flüssigen Phase im Sumpf verbleibt und der oder die Alkohole sowie gegebenenfalls vorhandene Leichtsieder gasförmig abgetrennt werden.

In einer besonderen Ausführungsform des erfindungsgemäßen Verfahrens ist es auch möglich, auf die Vakuumdestillation zu verzichten und die gesamte Überschussmenge an Alkohol und leichtsiedenden Nebenprodukten allein durch Strippen zu entfernen. Das Strippen kann diskontinuierlich, beispielsweise im Reaktionsgefäß, durchgeführt werden. Optional kann das Strippen auch kontinuierlich, beispielsweise in einer Füllkörperkolonne, durchgeführt werden. Der Gehalt an Restalkohol und leichtsiedenden Nebenprodukten kann auf diese Weise (allein durch Strippen) auf unter 500 ppm, bevorzugt unter 300 ppm, besonders bevorzugt unter 200 ppm abgesenkt werden.

Das erfindungsgemäße Verfahren zeichnet sich dadurch aus, dass weder vor noch während noch nach der Alkoholabtrennung eine Base zugesetzt wird. Nach der Abtrennung des Alkohols wird der verbleibende Ester, optional mit Zugabe eines Filterhilfsmittels, zur Abtrennung des Katalysators filtriert oder zentrifuguiert. Als Filtermedium können übliche Filter, wie beispielsweise Papierfilter, Filtertücher, polymere oder keramische Membranen, Metallsiebe, Verbundmembranen und/oder der Filterkuchen selbst eingesetzt werden. Zum Zentrifugieren können prinzipiell alle handelsüblichen Zentrifugen eingesetzt werden. Die Abtrennung des Katalysators erfolgt vorzugsweise bei einer Temperatur kleiner 160 °C, vorzugsweise im Temperaturbereich von 20 bis 150 °C, wobei eine Temperatur des Gemisches von 80 bis 130 °C bevorzugt ist. Besonders bevorzugt wird/werden aus dem Reaktionsgemisch nach Abtrennung des Alkohols der Katalysator bzw. dessen Folgeprodukt(e) ohne Basenbehandlung durch Filtration bei Temperaturen zwischen 100 und 130 °C abgetrennt. Schon bei diesen relativ hohen Temperaturen ist eine ausreichende Abtrennung des Katalysators durch Filtrierung möglich. Erst durch das erfindungsgemäße Verfahren konnte auf langwieriges Abkühlen auf Umgebungstemperatur vor der Katalysator-Abtrennung verzichtet werden.

Das nach Abtrennung des Katalysators erhaltene Produkt (Benzoesäureester oder Gemisch von zwei oder mehreren Benzoesäureestern) kann in hoher Reinheit erhalten werden. Der Gehalt an Benzoesäureester liegt, in Abhängigkeit von der Reinheit der Edukte, typischerweise bei über 99 %, bevorzugt über 99,5 %, besonders bevorzugt bei über 99,7 %. Der Gehalt an Zinnverbindungen, berechnet als metallisches Zinn, liegt unter 1 mg/kg (ppm), bevorzugt unter 0,1 mg/kg. Die Säurezahl des Produktes nach DIN EN ISO 2114 beträgt bevorzugt unter 0,1 mg KOH/g.

Nach Abtrennung des Katalysators durch Filtration ist das Reaktionsgemisch in der oben beschriebenen Reinheit erhältlich. Eine weitere Reinigung zur Abtrennung von gegebenenfalls vorhandenen höher siedenden Verunreinigungen durch fraktionierte Vakuumdestillation kann zwar optional erfolgen, ist jedoch in der Regel nicht notwendig.

Das erfindungsgemäße Verfahren kann in einem Behälter oder in mehreren hintereinander geschalteten Gefäßen, jeweils bevorzugt Rührreaktoren, durchgeführt werden. So kann beispielsweise die Veresterung und die Aufarbeitung in verschiedenen Gefäßen erfolgen.

Der bei der Aufarbeitung abgetrennte Alkohol, der gegebenenfalls Benzoesäureester und/oder Nebenprodukte enthalten kann, kann, gegebenenfalls nach Ausschleusung einer Teilmenge, für den nächsten Ansatz verwendet werden.

Mittels des erfindungsgemäßen Verfahrens sind Benzoesäureester aufweisende Zusammensetzungen erhältlich, die insbesondere Isononylbenzoat aufweisen können. Diese Zusammensetzungen können in Farben, Lacken, Klebstoffen oder Klebstoffkomponenten oder als Weichmacher für Kunststoffe, bevorzugt PVC, besonders bevorzugt in PVC-Plastisolen verwendet werden.

Die folgenden Beispiele sollen die Erfindung näher erläutern, ohne den Schutzbereich zu beschränken, der sich aus der Beschreibung und den Patentansprüchen ergibt.

### Beispiel 1: Herstellung von Isononylbenzoat mit Zinnoxalat als Katalysator (erfindungsgemäß)

Die Veresterung von Benzoesäure mit iso-Nonanol wurde in einem beheizbaren 4 Liter Dreihalskolben durchgeführt, der mit einem Rührer, Innenthermometer, Tauchrohr mit Probenahmestelle und Wasserabscheider mit aufgesetztem Kühler ausgestattet war. Über den Wasserabscheider mit Kühler konnte die Apparatur evakuiert werden. Der Wasserabscheider wurde mit iso-Nonanol gefüllt. In den Kolben wurden 915 g Benzoesäure (DSM Fine Chemicals, Gehalt > 99,9 %), 1296 g iso-Nonanol (OXENO Olefinchemie GmbH) und 0,55 g Zinnoxalat (Aldrich) vorgelegt und unter Rühren bis 210 °C erhitzt. Ab 210 °C wurde die Heizleistung reduziert, Temperatur und Rückfluss wurden über Vakuum konstant gehalten. Das im Wasserabscheider anfallende Wasser wurde abgetrennt, der anfallende Alkohol lief in den Reaktionskolben zurück. Wurde Wasser aus dem Wasserabscheider abgelassen, so wurde die entfernte Menge durch Zugabe von Reinalkohol ersetzt, um den Füllstand im Reaktionskolben konstant zu halten.

Der Veresterungsfortschritt wurde in regelmäßigen Abständen über die Säurezahl kontrolliert. Die Veresterung wurde beendet, als die Säurezahl kleiner 0,04 mg KOH/g betrug. Die Veresterungsdauer betrug hierfür ab Siedebeginn ca. 3 Stunden.

Zur Aufarbeitung wurde anstelle des Wasserabscheiders eine 10 cm Multifillkolonne mit aufgesetzter Claisenbrücke angebracht und der Überschussalkohol bei einer Temperatur von 180 °C und einem Druck von 10 mbar abdestilliert.

Zur Reinigung des so als Sumpfprodukt erhaltenen Rohesters wurde über das Tauchrohr Wasser (8 Massen-% bezogen auf die Menge an Rohester) eingedüst. Dabei wurde die Temperatur konstant bei 180 °C gehalten.

Nach Beendigung der Wasserdampfdestillation wurde der Ester unter Vakuum auf 100 °C abgekühlt und über eine Nutsche mit Filterpapier (Fa. Filtrak, mittel weitporig, Sorte 389) über einen 1,5 cm starken Filterkuchen, bestehend aus dem Filterhilfsmittel Filterperl D14 (Fa. Perlite), unter Vakuum gefiltert.

Der so erhaltene Ester war farblos (APHA 19, Farbzahl nach Hazen, gem. DIN ISO 6271) und klar und hatte eine Reinheit von 99,96 Massen-% (gaschromatographisch bestimmt) sowie eine Säurezahl von 0,01 mg KOH/g (bestimmt gemäß DIN EN ISO 2114). Der Restalkoholgehalt betrug 74 ppm (gaschromatographisch bestimmt). Der Zinngehalt betrug 10 µg/kg (ppb, bestimmt mittels Atomspektometrie (ICP-OES) gemäß DIN EN ISO 11885). Auch nach Abkühlen auf Raumtemperatur blieb der Ester klar.

### Beispiel 2: Herstellung von Isononylbenzoat mit Tetrabutyltitanat als Katalysator (ohne Neutralisation, Vergleichsbeispiel)

Die Veresterung von Benzoesäure mit iso-Nonanol wurde in einem beheizbaren 4 Liter Dreihalskolben durchgeführt, der mit einem Rührer, Innenthermometer, Tauchrohr mit Probenahmestelle und Wasserabscheider mit aufgesetztem Kühler ausgestattet war. Über den Wasserabscheider mit Kühler konnte die Apparatur evakuiert werden. Der Wasserabscheider wurde mit iso-Nonanol (OXENO Olefinchemie GmbH) gefüllt. In den Kolben wurden 915 g Benzoesäure (DSM Fine Chemicals, Gehalt >99,9 %), 1296 g iso-Nonanol und 0,55 g Tetra-n-Butyltitanat (FLUKA) vorgelegt und unter Rühren bis 210 °C erhitzt. Ab 210 °C wurde die Heizleistung reduziert, Temperatur und Rückfluss wurden über Vakuum konstant gehalten. Das im Wasserabscheider anfallende Wasser wurde abgetrennt, der anfallende Alkohol lief in den Reaktionskolben zurück. Wurde Wasser aus dem Wasserabscheider abgelassen, so wurde die entfernte Menge durch Zugabe von Reinalkohol ersetzt, um den Füllstand im Reaktionskolben konstant zu halten.

Der Veresterungsfortschritt wurde in regelmäßigen Abständen über die Säurezahl kontrolliert. Die Veresterung wurde beendet, als die Säurezahl unterhalb von 0,04 mg KOH/g war. Die Veresterungsdauer betrug ab Siedebeginn ca. 2,5 Stunden.

Zur Aufarbeitung wurde anstelle des Wasserabscheiders eine 10 cm Multifillkolönne mit aufgesetzter Claisenbrücke angebracht und der Überschussalkohol bis zu einer Temperatur von 180 °C bei 10 mbar abdestilliert.

Zur Reinigung des so erhaltenen Rohesters wurde über das Tauchrohr Wasser (8% bezogen auf die Menge an Rohester) eingedüst. Dabei wurde die Temperatur konstant bei 180 °C gehalten.

Nach Beendigung der Wasserdampfdestillation wurde der Ester unter Vakuum auf 100 °C abgekühlt und über eine Nutsche mit Filterpapier (Fa. Filtrak, mittel weitporig, Sorte 389) über einen 1,5 cm starken Filterkuchen, bestehend aus dem Filterhilfsmittel Filterperl D14 (Fa. Perlite), unter Vakuum gefiltert.

Der so erhaltene Ester war farblos (APHA 19; (Farbzahl nach Hazen, gem. DIN ISO 6271) und klar und hatte eine Reinheit von 99,96 % (gaschromatographisch bestimmt), allerdings eine deutlich erhöhte Säurezahl von 0,12 mg KOH/g. Der Restalkoholgehalt lag bei 197 ppm (gaschromatographisch bestimmt). Der Titangehalt lag bei 33 mg/kg (bestimmt mittels Atomspektometrie (ICP-OES) gemäß DIN EN ISO 11885). Bei Abkühlen auf Raumtemperatur wurde der Ester trüb. Nur durch zusätzliche Reinigungsschritte wie z.B. waschen, ablassen des Waschwassers und erneutes aufheizen zum Trocknen konnte ein Produkt erhalten werden, das eine Säurezahl von < 0,05 mg KOH/g, einen Titangehalt von <0,1 mg/kg hatte und auch nach Abkühlen auf Raumtemperatur klar blieb.

### Beispiel 3: Herstellung von Isononylbenzoat mit Tetrabutyltitanat als Katalysator (mit Neutralisation, Vergleichsbeispiel)

Die Veresterung von Benzoesäure mit iso-Nonanol (OXENO Olefinchemie GmbH) wurde in einem beheizbaren 4 Liter Dreihalskolben durchgeführt, der mit einem Rührer, Innenthermometer, Tauchrohr mit Probenahmestelle und Wasserabscheider mit aufgesetztem Kühler ausgestattet war. Über den Wasserabscheider mit Kühler konnte die Apparatur evakuiert werden. Der Wasserabscheider wurde mit iso-Nonanol gefüllt. In den Kolben wurden 915 g Benzoesäure (DSM Fine Chemicals, Gehalt >99,9 %), 1296 g iso-Nonanol und 0,55 g Tetra-n-Butyltitanat (FLUKA) vorgelegt und unter Rühren bis 210 °C erhitzt. Ab 210 °C wurde die Heizleistung reduziert, Temperatur und Rückfluss wurden über Vakuum konstant gehalten. Das im Wasserabscheider anfallende Wasser wurde abgetrennt, der anfallende Alkohol lief in den Reaktionskolben zurück. Wurde Wasser aus dem Wasserabscheider abgelassen, so wurde die entfernte Menge durch Zugabe von Reinalkohol ersetzt, um den Füllstand im Reaktionskolben konstant zu halten.

Der Veresterungsfortschritt wurde in regelmäßigen Abständen über die Säurezahl kontrolliert. Die Veresterung wurde beendet, als die Säurezahl unter 0,1 mg KOH/g war. Die Veresterungsdauer betrug ab Siedebeginn ca. 2 Stunden.

Danach wurde auf 80 °C abgekühlt und mit dem 6 fachen der aus der Säurezahl bestimmten stöchiometrisch notwendigen Menge an wässriger Natronlauge (5 Gew.-%) neutralisiert. Anschließend wurde noch ca. 30 Minuten bei dieser Temperatur gerührt.

Zur Aufarbeitung wurde anstelle des Wasserabscheiders eine 10 cm Multifillkolonne mit aufgesetzter Claisenbrücke angebracht, das Produkt auf 180 °C aufgeheizt und der Überschussalkohol bis zu einer Temperatur von 180 °C bei 10 mbar abdestilliert.

Zur Reinigung des so erhaltenen Rohesters wurde über das Tauchrohr Wasser (8% bezogen auf die Menge an Rohester) eingedüst. Dabei wurde die Temperatur konstant bei 180 °C gehalten.

Nach Beendigung der Wasserdampfdestillation wurde der Ester unter Vakuum auf 100 °C abgekühlt und über eine Nutsche mit Filterpapier (Fa. Filtrak, mittel weitporig, Sorte 389) über einen 1,5 cm starken Filterkuchen, bestehend aus dem Filterhilfsmittel Filterperl D14 (Fa. Perlite), unter Vakuum gefiltert.

Der so erhaltene Ester war farblos (APHA 14; Farbzahl nach Hazen, gem. DIN ISO 6271) und zunächst klar und hatte eine Reinheit von 99,98 % (gaschromatographisch bestimmt), eine Säurezahl von 0,01 mg KOH/g. Der Restalkoholgehalt lag bei 104 ppm (gaschromatographisch bestimmt).

Nach dem Abkühlen auf Raumtemperatur fiel allerdings ein Niederschlag aus, der eine weitere Aufarbeitung (z. B. Filtration) notwendig machte. Der nach der Aufarbeitung bestimmte Titangehalt lag unterhalb der Nachweisgrenze von 0,1 mg/kg (bestimmt mittels Atomspektometrie (ICP-OES) gemäß DIN EN ISO 11885).

Wie an Hand der Beispiele 1 bis 3 leicht zu erkennen ist, ist es mit dem erfindungsgemäßen Verfahren auf einfache Weise ohne Neutralisation und ohne einen zusätzlichen Aufarbeitungsschritt möglich, einen Benzoesäureester herzustellen, der einen Metallgehalt von kleiner 1 mg/kg aufweist.

### Beispiel 4: Herstellung von Isotridecylbenzoat (erfindungsgemäß)

Die Veresterung von Benzoesäure mit iso-Tridecanol wurde in einem beheizbaren 4 Liter Dreihalskolben durchgeführt, der mit einem Rührer, Innenthermometer, Tauchrohr mit Probenahmestelle und Wasserabscheider mit aufgesetztem Kühler ausgestattet war. Der Wasserabscheider wurde mit iso-Tridecanol (OXENO Olefinchemie GmbH) gefüllt. In den Kolben wurden 732 g Benzoesäure (DSM Fine Chemicals, Gehalt >99,9 %), 1600 g iso-Tridecanol und 0,44 g Zinnoxalat (Aldrich) vorgelegt und unter Rühren bis 210 °C erhitzt. Ab 210 °C wurde die Heizleistung reduziert, Temperatur und Rückfluss wurden über Zugabe von Toluol konstant gehalten. Das im Wasserabscheider anfallende Wasser wurde abgetrennt, das anfallende Alkohol / Toluol Gemisch lief in den Reaktionskolben zurück. Wurde Wasser aus dem Wasserabscheider abgelassen, so wurde die entfernte Menge durch Zugabe von Reinalkohol ersetzt. Der Veresterungsfortschritt wurde in regelmäßigen Abständen über die Säurezahl kontrolliert.

Die Veresterung wurde beendet, als die Säurezahl kleiner 0,04 mg KOH/g war. Die Veresterungsdauer betrug hierfür ab Siedebeginn 3 Stunden.

Zur Aufarbeitung wurde anstelle des Wasserabscheiders eine 10 cm Multifillkolonne mit aufgesetzter Claisenbrücke angebracht und der Überschussalkohol bis 210 °C, 10 mbar abdestilliert.

Zur Reinigung des so erhaltenen Rohesters wurde über das Tauchrohr Wasser (8% bezogen auf die Menge an Rohester) eingedüst. Dabei wurde die Temperatur konstant bei 180 °C gehalten.

Nach Beendigung der Wasserdampfdestillation wurde der Ester unter Vakuum auf 100 °C abgekühlt und über eine Nutsche mit Filterpapier (Fa. Filtrak, mittel weitporig, Sorte 389) über einen 1,5 cm starken Filterkuchen, bestehend aus dem Filterhilfsmittel Filterperl D14 (Fa. Perlite), unter Vakuum gefiltert.

Der so erhaltene Ester war klar und farblos (APHA = 15; Farbzahl nach Hazen; gem. DIN ISO 6271). Die Reinheit betrug 99,97 % (gaschromatographisch bestimmt) bei einem Restalkoholgehalt von 280 ppm (gaschromatographisch bestimmt). Die Säurezahl des Esters lag bei 0,026 mg KOH/g. Der Zinngehalt liegt unter 1 mg/kg (bestimmt mittels Atomspektometrie (ICP-OES) gemäß DIN EN ISO 11885).

## Patentansprüche

1. Verfahren zur Herstellung von Benzoesäureestern, deren Alkoxygruppen 9 oder 13 Kohlenstoffatome aufweisen, durch Umsetzung von Benzoesäure mit zumindest einem Alkohol, der 9 oder 13 Kohlenstoffatome aufweist, wobei das entstehende Reaktionswasser während der Veresterungsreaktion durch Destillation mit dem im Überschuss eingesetzten Alkohol aus dem Reaktionsgemisch entfernt wird und der bei der Veresterungsreaktion nicht umgesetzte Alkohol nach der Veresterungsreaktion entfernt wird,
**dadurch gekennzeichnet,**
**dass** die Umsetzung in Gegenwart einer Zinn (II) Verbindung als Katalysator bei einer Temperatur von 160 bis 250 °C erfolgt und dass ohne eine Behandlung mit einer Base aus dem nach der Abtrennung des nicht umgesetzten Alkohols verbleibenden Reaktionsgemisch der Katalysator und/oder dessen zinnhaltige Folgeprodukte durch eine Filtration oder durch Zentrifugieren so weit abgetrennt wird, dass der Zinngehalt des Endproduktes unter 1 mg/kg liegt und dass als Alkohole Nonanole und/oder Tridecanole eingesetzt werden.

2. Verfahren nach Anspruch 1,
**dadurch gekennzeichnet,**
**dass** ein Gemisch von Alkoholen mit gleicher oder unterschiedlicher Anzahl an Kohlenstoffatomen eingesetzt wird.

3. Verfahren nach Anspruch 2, **dadurch gekennzeichnet, dass** zu Beginn der Reaktion ein molares Verhältnis von Zinn zu Benzoesäure in einem Bereich von 10 ppm zu 1 bis 1000 ppm zu 1 eingestellt wird.

4. Verfahren nach Anspruch 3, **dadurch gekennzeichnet, dass** die Benzoesäure bis zu einer Säurezahl von kleiner als 0,1 mg KOH/g, bestimmt nach DIN EN ISO 2114, umgesetzt wird.

5. Verfahren nach Anspruch 4,**dadurch gekennzeichnet,**
**dass** die Abtrennung des Katalysators bei einer Temperatur unterhalb von 160 °C erfolgt.

6. Verfahren nach Anspruch 5, **dadurch gekennzeichnet,**
**dass** aus dem Reaktionsgemisch nach Abtrennung des Alkohols der Katalysator bzw. dessen Folgeprodukt(e) ohne Basenbehandlung bei Temperaturen unter 130 °C abgetrennt wird/werden.

7. Verfahren nach Anspruch 6, **dadurch gekennzeichnet, dass** als Filtermedium eine polymere oder keramische Membran, Verbundmembran oder ein Papierfilter eingesetzt wird.

## Claims

1. Process for preparing benzoic esters whose alkoxy groups have 9 or 13 carbon atoms by reacting benzoic acid with at least one alcohol having 9 or 13 carbon atoms, the water of reaction formed being removed from the reaction mixture during the esterification reaction by distillation with the alcohol used in excess, and the alcohol not converted in the esterification reaction being removed after the esterification reaction,
**characterized**
**in that** the reaction takes place in the presence of a tin(II) compound as catalyst at a temperature of 160 to 250°C and in that, without treatment with a base, the catalyst and/or its tincontaining derivatives are removed by filtering or by centrifuging from the reaction mixture which remains after the unconverted alcohol has been separated off, to an extent such that the tin content of the end product is below 1 mg/kg and in that alcohols used are nonanols and/or tridecanols.

2. Process according to Claim 1,
**characterized**
**in that** a mixture of alcohols with the same or different number of carbon atoms is used.

3. Process according to Claim 2, **characterized**
**in that** a molar ratio of tin to benzoic acid in a range of from 10 ppm : 1 to 1000 ppm : 1 is set at the beginning of the reaction.

4. Process according to Claim 3, **characterized**
**in that** the benzoic acid is reacted to an acid number of less than 0.1 mg KOH/g, determined in accordance with DIN EN ISO 2114.

5. Process according to Claim 4, **characterized**
**in that** the catalyst is separated off at a temperature below 160°C.

6. Process according to Claim 5, **characterized**
**in that** the catalyst and/or derivative(s) thereof is/are separated off from the reaction mixture, after the alcohol has been separated off and without base treatment, at temperatures below 130°C.

7. Process according to Claim 6, **characterized**
**in that** a polymeric or ceramic membrane, composite membrane or paper filter is used as filter medium.

## Revendications

1. Procédé de fabrication d'esters de l'acide benzoïque dont les groupes alcoxy comprennent 9 ou 13 atomes de carbone, par mise en réaction d'acide benzoïque avec au moins un alcool qui comprend 9 ou 13 atomes de carbone, l'eau de réaction formée étant éliminée du mélange réactionnel par distillation pendant la réaction d'estérification avec l'alcool utilisé en excès et l'alcool non réagi pendant la réaction d'estérification étant éliminé après la réaction d'estérification,
**caractérisé en ce que**
la réaction a lieu en présence d'un composé d'étain (II) en tant que catalyseur à une température de 160 à 250 °C, et **en ce que** le catalyseur et/ou ses produits dérivés contenant de l'étain sont éliminés du mélange réactionnel restant après la séparation de l'alcool non réagi sans traitement avec une base par filtration ou centrifugation de manière à ce que la teneur en étain du produit final soit inférieure à 1 mg/kg, et **en ce que** des nonanols et/ou des tridécanols sont utilisés en tant qu'alcools.

2. Procédé selon la revendication 1, **caractérisé en ce qu'**un mélange d'alcools contenant un nombre identique ou différent d'atomes de carbone est utilisé.

3. Procédé selon la revendication 2, **caractérisé en ce qu'**un rapport molaire entre l'étain et l'acide benzoïque dans une plage allant de 10 ppm sur 1 à 1 000 ppm sur 1 est ajusté au début de la réaction.

4. Procédé selon la revendication 3, **caractérisé en ce que** l'acide benzoïque est transformé jusqu'à un indice d'acidité inférieur à 0,1 mg KOH/g, déterminé selon DIN EN ISO 2114.

5. Procédé selon la revendication 4, **caractérisé en ce que** la séparation du catalyseur a lieu à une température inférieure à 160 °C.

6. Procédé selon la revendication 5, **caractérisé en ce que** le catalyseur ou son ou ses produits dérivés sont séparés du mélange réactionnel après séparation de l'alcool sans traitement avec une base à des températures inférieures à 130 °C.

7. Procédé selon la revendication 6, **caractérisé en ce qu'**une membrane polymère ou céramique, une membrane composite ou un filtre en papier est utilisé en tant que moyen de filtration.
